# EUROPEAN PATENT SPECIFICATION

(11) **EP 1 212 023 B1**
(45) Date of publication and mention of the grant of the patent: **28.09.2005**
(21) Application number: 01941745.0
(22) Date of filing: 31.05.2001
(51) Int. Cl.: A61F 11/00

(54) **PROCESS FOR APPLYING A DECORATIVE PATTERN TO EARPLUGS**
VERFAHREN ZUM AUFBRINGEN EINES DEKORATIVEN MUSTERS AUF OHRSTÖPSELN
PROCEDE SERVANT A APPLIQUER UN MOTIF DECORATIF A DES BOUCHONS D'OREILLE

(30) Priority: 31.05.2000 US 208250 P
(43) Date of publication of application: 12.06.2002
(73) Proprietor: Cabot Safety Intermediate Corporation, Southbridge, MA 01550 (US)
(72) Inventor: MAUDE, Daniel, A., Carmel, IN 46032 (US); JURNEY, John, Carmel, IN 46032 (US)
(74) Representative: Reinhardt, Harry
(86) International application number: PCT/US2001/017641
(87) International publication number: WO 2001/091680

(56) References cited:
- US-A- 4 713 843
- US-A- 5 881 729
- US-A- 5 940 886
- US-A- 6 058 516

## Description

Environmental sounds typically include a mixture of various sound wave frequencies having varying intensities. It is well documented that repeated or prolonged exposure to sounds of sufficiently high sound pressure level will cause temporary or permanent hearing loss. For example, exposure to sound waves of some frequencies and of varying intensities under prolonged exposure can damage the auditory organ and cause serious hearing problems, including deafness. Injurious noises such as those caused by explosions or bursts often include a mixture of sound wave frequencies of varying intensity. These disturbing frequencies are in both the high and low frequency bands and have an intensity sufficient to cause hearing problems. Individuals who are frequently exposed to sound having such disturbing and sometimes dangerous frequencies and intensities run the risk of incurring such injuries as hearing loss or even deafness. These individuals include workers at demolition or construction sites, operators of heavy, noisy equipment and those in actiye military service. Ear (i.e., hearing) protection is needed to prevent a loss in hearing acuity and the gradual increase in the threshold of hearing resulting from extended exposures to loud noise. Sound attenuation devices are known which specifically address this problem. These include earplugs, earmuffs, and the like which function to reduce the negative effects of exposure to dangerous frequencies by limiting the entry of all sound waves into the auditory organ.

Earplugs may be with a base color. Earplugs may also have multiple colors. One method of forming an earplug having multiple colors is to cast in succession the earplug so that individual colored sections are formed. Another method of manufacture comprises molding the earplug with a predetermined number of colors. However, these processes for coloring the earplugs are complex and costly due to the use of multiple streams and/or molds.

In another method, multi-coloured earplugs may be formed by mixing colours during the formation of the earplugs. One of the associated disadvantages of this method is that the colouring is not consistent from one earplug to another. In other words, the colouring is often random from one earplug to another earplug and this results in a lack of consistency from one earplug to another earplug. The pattern is therefore not consistently repeated in precise detail from one earplug to another earplug.

US-A 5,881,729 describes an earplug which may be constructed of a material having various colorations and patterns to be visible if it should become unattached from the user. Thus, it shows constructing an earplug of a certain material such that colorations or patterns appear thereon.

US-A 5 153 387 discloses an earplug made of polymeric foam and a method of producing it.

US-A 4,713,843 describes an ear protector worn over the ear for keeping the ear warm. An outer surface of an outer protection layer is configured so that it can be imprinted with a message or the like. Thus, it discloses only that an outer surface of an ear-warmer may be imprinted.

The above-discussed and other drawbacks and deficiencies are overcome or alleviated by a process of producing an earplug and forming a pattern as claimed. An undecorated earplug made of polymeric foam is delivered to a printing device which is designed to apply a pattern to one or more sections of the earplug.

In the exemplary embodiments, pattern may be formed of a custom logo(s); printed text, such as a company name or product name; decorative patterns and other patterns used for graphic styling or earplug identification. In another exemplary embodiment the pattern may be applied along the length of the earplug as well as on either end of the earplug. The pattern may further be formed of any number of colors and is not limited to a specific or single color.

In another exemplary embodiment, the undecorated earplug is conveyed to a printing station which includes the printing device and further optionally includes an alignment device which serves to orient the earplug relative to the printing device. The delivery of the undecorated earplug as well as the printing device may be part of an automated system which offers high speed performance and is designed to pattern the earplugs so that the pattern is repeatable and consistent from earplug to earplug. The earplug may be oriented so that the pattern may be applied to any one of the sides of the earplug as well as to either of the end surfaces of the earplug. Any number of printing devices may be used so long as the printing device is designed to provide an effective transfer of the pattern onto the earplug.

According to one embodiment, the printing device comprises an ink jet printer and the printing process is characterized as a non-impact printing process. During this non-impact printing process, the earplug is not deformed by the printer but rather is oriented so that the pattern is applied to the preselected, specific areas of the earplug

By using this or a similar type of printing device, the pattern may be repeated over and over with precision. Therefore, the appearance of the earplug will not significantly vary from one earplug to another earplug. This type of printing process is also cost effective and simple and produces earplugs having consistent patterns formed thereon.

The above-discussed and other features and advantages of the present invention will be appreciated and understood by those skilled in the art from the following detailed description and drawings.

### BRIEF DESCRIPTION OF THE DRAWINGS

Referring now to the drawings wherein like elements are numbered alike in the several FIGURES:
FIGURE 1 is a perspective view of an earplug according to a first embodiment showing a first graphic design;
FIGURE 2 is a perspective view of an earplug according to a second embodiment showing a second graphic design;
FIGURE 3 is a perspective view of an earplug according to a third embodiment showing a third graphic design;
FIGURE 4 is a perspective view of an earplug according to a fourth embodiment showing a fourth graphic design;
FIGURE 5 is a perspective view of an earplug according to a fifth embodiment showing a fifth graphic design;
FIGURE 6 is a perspective view of an earplug according to a sixth embodiment showing a sixth graphic design;
FIGURE 7 is a general schematic drawing outlining a printing process according to the present invention; and
FIGURE 8 is a perspective partially cut-away view of an exemplary nozzle assembly for transferring ink onto a surface of the earplug to form the pattern.

### DETAILED DESCRIPTION OF THE PREFERRED EMBODIMENT

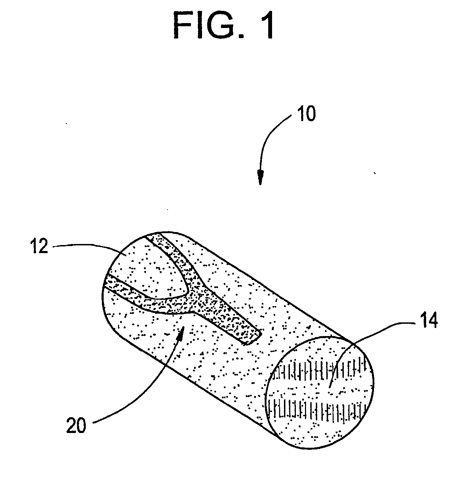

Referring now to FIGURE 1 in which an exemplary earplug construction according to a first embodiment is illustrated and generally indicated at 10. The earplug 10 may have any number of shapes and sizes and is formed from suitable materials which are designed for earplug construction. The earplug 10 has a first end 12 and an opposing second end 14. The earplug 10 has a predetermined first pattern (first graphic design) formed thereon and generally indicated at 20. The first pattern 20 may comprise any number of color patterns and be formed of only one color or be formed of a multitude of colors. In the illustrated embodiment, the first pattern 20 resembles a flame and is formed such that the first pattern 20 is visually distinct from the remaining unpatterned portions of the earplug 10. For example, the earplug 10 is formed of a base color and the first pattern 20 is formed of at least one second color which is visually distinct from the base color. When it is desired for the first pattern 20 to resemble a flame-like graphic display, the first pattern 20 may be formed of a red or orange color and the base color is yellow.

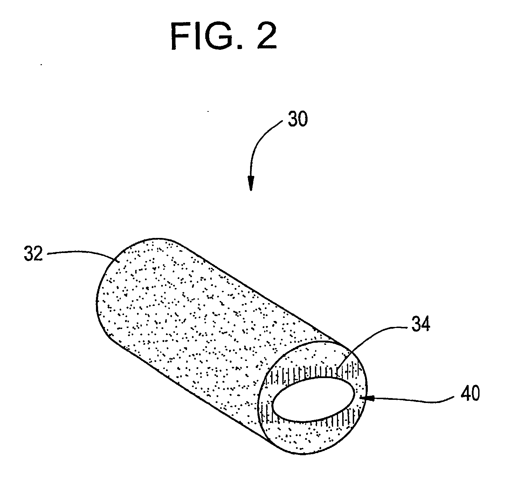

Now referring to Figure 2 in which an earplug 30 according to a second embodiment is shown. In this embodiment, the earplug 30 has a first end 32 and an opposing second end 34. The earplug 30 has a second pattern 40 formed thereon. In the illustrated embodiment, the second pattern 40 is formed at one of the first and second ends 32, 34 and comprises an indicia display area in which a logo or the like may be formed and will be visually apparent to users and other individuals. When it is intended for the second pattern 40 to comprise an indicia display area for displaying a logo, the second pattern 40 may take any number of shapes and sizes. In the illustrated embodiment, the second pattern 40 has an oval shape in which the logo (not shown) or any other symbol or character(s) may be provided. The earplug 30 will likely have a base color and the second pattern 40 will preferably be formed of a second visually distinct color in comparison to the base color. For example, the second pattern 40 may have a border formed of one color and then the logo printed therein is formed of another color. The base color is preferably yellow. It is further understood that the second pattern 40 may also be formed on other locations of the earplug 30 besides at one of the first and second ends 32, 34. For example, the second pattern 40 may be formed on the earplug 30 between the first and second ends 32, 34.

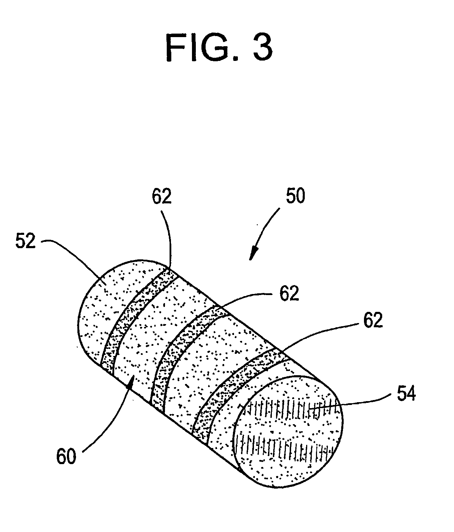

FIGURE 3 shows an earplug 50 according to a third embodiment. Earplug 50 has a first end 52 and an opposing second end 54. Earplug 50 further has a third pattern 60 formed thereon. The illustrated third pattern 60 is formed of a plurality of stripes 62 which extend circumferentially around the earplug 50. The earplug 50 is formed of a base color and the plurality of stripes 62 are formed of a color which is visually distinct from the base color. It will be appreciated that the plurality of stripes 62 may be formed of one or more colors so long as each color is visually distinct from the base color. Each stripe 62 may have a predetermined width and the spacing between stripes 62 will also accordingly be variable. In the illustrated embodiment, there are three stripes 62 which extend around the earplug 50, wherein next adjacent stripes 62 are parallel to each other. The base color is preferably yellow.

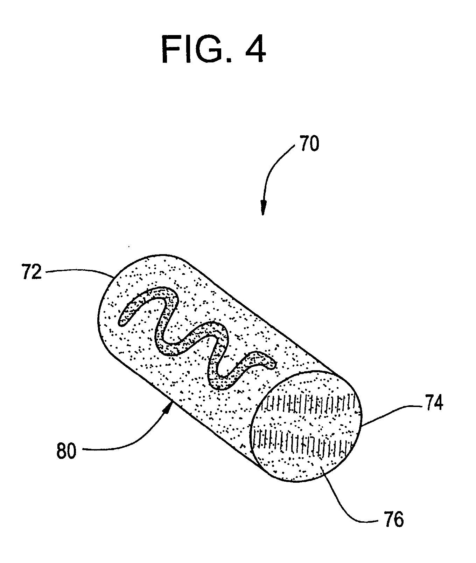

FIGURE 4 shows an earplug 70 according to a fourth embodiment. Earplug 70 has a first end 72 and an opposing second end 74. The earplug 70 is generally cylindrical in nature and therefore includes first and second end surfaces 76. The earplug 70 has a fourth pattern 80 formed thereon. The fourth pattern 80 comprises a swirl-like pattern formed of a color which is visually distinct from a base color of the earplug 70. The base color is preferably yellow. It will be understood that the illustrated fourth pattern 80 is merely exemplary in nature and any number of patterns may be used to provide a design to the earplug 70. The width of the colored markings which form the fourth pattern 80 may be varied depending upon the application and to create a different visual look for the earplug 70.

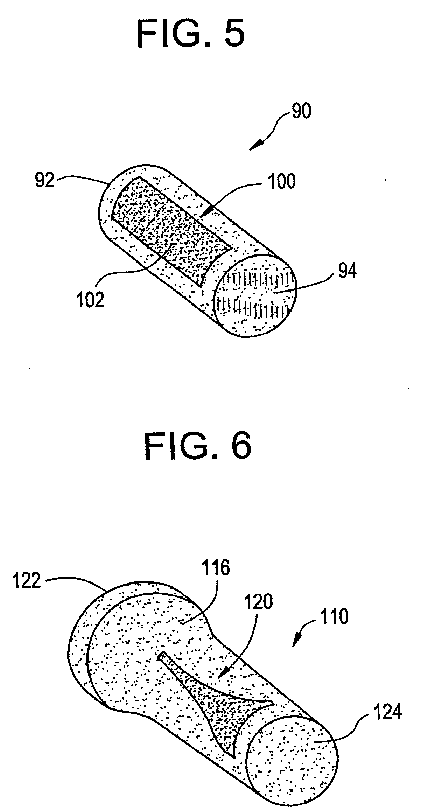

FIGURE 5 is a perspective view of an earplug 90 according to a fifth embodiment. In this embodiment, the earplug 90 has a fifth pattern 100 formed thereon and as illustrated, the fifth pattern 100 comprises a predetermined number of longitudinal stripes 102 which extend between a first end 92 and an opposing second end 94 of the earplug 90. The longitudinal stripes 102 are spaced circumferentially around the earplug 90. In the illustrated embodiment, each longitudinal stripe 102 has a generally rectangular shape and it will be appreciated that the width of the longitudinal stripe 102 may be varied which thereby varies the distance between next adjacent longitudinal stripes 102. The earplug 90 is formed of a base color and the fifth pattern 100 is formed of a visually distinct color relative to the base color.

FIGURE 6 is a perspective view of an earplug 110 according to a sixth embodiment. In this sixth embodiment, the earplug 110 has a sixth pattern 120 formed thereon. The exemplary sixth pattern has a first end 122 and an opposing second end 124 with the second end 124 having a width greater than a width of the pattern 120 at the first end 122. Thus, the sixth pattern 120 presents a design which tapers from the second end 124 to the first end 122. The earplug 110 is also slightly different than the earplugs of the other embodiments in that the earplug 110 has a first end 112 and an opposing second end 114 with a tapered section 116 being formed therebetween. The first end 112 thus has a greater diameter than a diameter at the second end 114 and the first end 112 is intended to be a free end which is grasped by a user for insertion and removal of the earplug 110 into an ear of the user. The opposing second end 114 is intended to comprise an insertion end which is inserted into the ear of the user.

It will be understood that the above-described and illustrated patterns (graphic patterns) are merely exemplary in nature and that any number of patterns may be formed on the earplug including providing graphic design's decoration and characters which serve to identify the particular individual earplug. It will also be appreciated that the particular shape and size of the earplug may be varied depending upon the particular application.

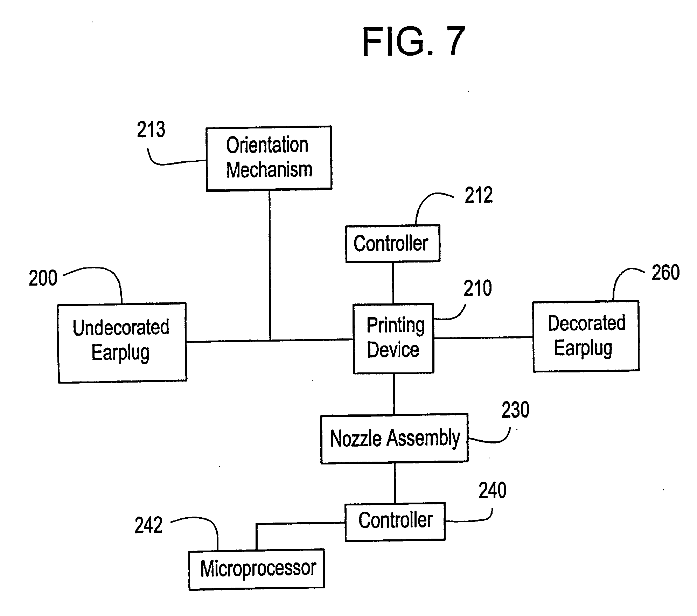

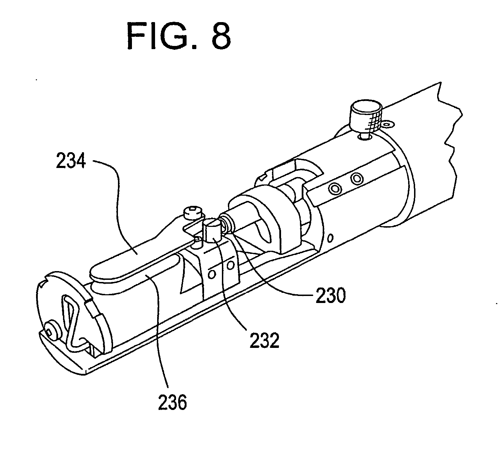

Referring to FIGURES 7-8, an exemplary process for coloring earplugs is described. Broadly, the exemplary process comprises orienting and conveying unpatterned earplugs 200 past a high-speed printing device 210. Such an exemplary process, wherein the earplugs 200 may be delivered and transferred from the printing device 210 through a high-speed automation assembly, provides a pattern that is repeatable and consistent from earplug to earplug. (The printing device 210 may also be designed so that it may be aligned relative to the earplug 200 so that all sides and the ends of the earplug 200 may be printed on by the printing device 210.)

The printing device 210 may comprise any number of suitable printing devices which are designed to effectively print a pattern on a surface of the earplug. The printing device 210 may comprise an impact type printing device or a non-impact printing type device in which the printing device 210 does not contact the earplug. Suitable impact type printing processes in which the printing device 210 at least makes partial contact with the earplug during the print transfer are PAD based printing processes and a heat transfer printing process, as will be described in greater detail hereinafter. Suitable non-impact printing processes include but are not limited to laser printing processes, ink jet printing processes and dot matrix type printing processes. In one exemplary embodiment, the printing device 210 comprises an ink jet printer. One particularly suitable ink jet printing device 210 is commercially available from Willett under the trade name "460 Si Continuous Ink Jet (CIJ)". This printing device 210 is designed to produce high quality print on a wide range of products and according to the present invention is used to produce the pattern or graphic display on the earplug 200. The printing device 210 includes a controller 212 which is operated with software so that a user may program and monitor the printing device 210 and defme and store desired patterns and graphic displays for application onto the earplug 200. A suitable ink jet printer 210, such as the one identified above, operates by supplying ink under pressure to a nozzle assembly 230 where the ink is forced out through a small orifice. The nozzle assembly 230 is vibrated causing the ink jet to break up into a continuous stream of small droplets. These small droplets pass through a charge electrode 232 where an electrical charge is applied to the droplets. The charge is varied depending upon where the droplet is to positioned on the product substrate. A microprocessor control system monitors the feedback from the phase detector to check that droplet charging is synchronised to droplet break up. The charged droplets then pass through two deflector electrodes 234, 236 which have a contact voltage difference applied between them. As droplets pass through the electrical field produced between the electrodes 234, 236, the droplets are reflected from their original path by an amount proportional to their charge. The deflected drop will then strike the substrate and by varying the charge on consecutive drops, a predetermined column can be printed. As the substrate moves the further columns can be printed to form characters or graphics. It will be appreciated that the printing device 210 is preferably fitted and programmed so that color printing is available. It being understood that other types of printing devices may be used to form the pattern on the earplug and the present invention is not limited to the above-described printing device which is merely exemplary in nature. The printing device 210 may be designed so that one printing device 210 serves to print a single color on the earplug 200. Thus, it will be appreciated that multiple printing devices 210 maybe used to provide a multi-colored decorative pattern.

In one aspect, the earplugs 200 may be delivered to the printing device 210 using conventional transportation mechanisms including using conveyor systems. During a non-impact printing process, the earplugs 200 do not contact the printing device 210 and therefore, the earplugs 200 do not deform as a result of pressure being exerted on the earplugs 200 by the device 210 itself. This permits the earplugs 200 to advantageously maintain their form during the printing process. Once the earplugs 200 are delivered to the printing device 210, the earplugs 200 may be properly positioned relative to the printing device 210 so that the printing device 210 has access to all sides of the earplug 200 and also to the end surfaces of the earplug 200 for forming the pattern thereon. An orientation mechanism, generally indicated at 213, may be used to position the earplug 200 relative to the nozzle assembly 230 and manipulate the earplug 200 so that various sides are positioned relative to the printing device 210 to permit the pattern to be applied thereon. It will be appreciated that the nozzle assembly 230, which disperses the ink, may be coupled to a controller 240 which is designed to control movement of the nozzle assembly 230. Thus, depending upon the precise application, the nozzle assembly 230 may be moved to a first position for forming a graphic or other printed pattern on a first portion of the earplug 200 and then the controller 240 causes the nozzle assembly 230 to be moved to a second position relative to the earplug 200 where the same or different graphic or other printed pattern is applied to the surface of the earplug 200.

In another aspect, the controller 240 may be connected to a microprocessor 242 and computer unit which serves to drive the nozzle assembly 230 to predetermined sections of the earplug 200 as a result of user inputted information. The computer unit may be designed to store the precise shape of the earplug 200 being used and also the computer unit stores a number of preselected graphic designs and other printed patterns. Based on the information the user inputs and more specifically, based upon the type of earplug being used and the selected graphic pattern, the nozzle assembly 230 and more generally, the printing device 210 may be moved relative to the earplug 200 by the controller 212 and the computer unit so that the selected graphic pattern (or other printed pattern) may be applied to the earplug at specific sections thereof. The nozzle assembly 230 may be disposed in a shoot or the like and positioned above the earplug 200. The shoot serves to fix the nozzle assembly 230 relative to the earplug 200.

In another aspect, it will also be appreciated that the earplug 200 may be delivered to a printing station which houses the printing device 210. At the printing station, the earplug 200 may be manipulated so that any one of its sides and/or end surfaces face the printing device 210 for application of the graphic pattern or other printed pattern on selected portions of the earplug 200. In this configuration, the nozzle assembly 230 is more stationary than the previous printing configuration and the earplug 200 may be adjusted relative to the nozzle assembly 230 so that selected portions of the earplug 200 face the nozzle assembly 230 for application of graphic pattern thereto.

In another aspect, it will also be appreciated that the printing device 210 may actually comprise more than one printing device which are spaced relative to one another and are orientated so that patterns are applied to selected sections of the earplug 200. For example, in one embodiment three printing devices 210 may be provided and spaced generally 120° apart from one another. When the earplug 200 is positioned relative to the printing device 210, the three printing devices 210 may be concentrated on three sections of the earplug 200 which are each 120° apart from one another. Thus, the actuation of the printing devices 210 results in three sections of the earplug 200 being printed on and thus, three separate patterns are formed on the earplug 200. The patterns may be the same pattern or may be different patterns. In this embodiment, it may not be necessary to move the earplug 200 relative to the printing devices 210 because the printing devices 210 have already been properly positioned and orientated to provide the patterns on the earplug 200. In another embodiment, four printing devices 210 may be provided are spaced at predetermined angles relative to one another. For example, the four printing devices 210 maybe orientated in a 90° pattern so that a 90° angle is formed between each printing device 210. In this design, the patterns may be thus disposed on the earplug 200 so that each pattern is orientated 90° relative to another pattern. It will be understood that any number of printing devices 210 may be used to provide the desired pattern appearance on the earplug.

After the graphic pattern has been applied to the earplug 200, the resulting earplug, generally indicated at 260, may then be transferred away from the printing station to another location where the earplugs 260 may be packaged and/or further processed.

It will be further appreciated by one of skill in the art that other types of printing devices 210 and printing processes may be used according to the present invention. For example, an impact type printing process involving a heat transfer printing process (not shown) which is suitable for transferring a decorative graphic pattern onto the surface of the earplug may be used according to the present invention. In a heat transfer printing process, a printing medium containing the graphic pattern may be applied to the earplug so that a print transfer side of the printing medium faces and is in contact with the earplug. Subsequently, heat may be applied to an opposite side of the printing medium causing the graphic pattern contained in the printing medium to be transferred from the printing medium to a surface of the earplug. This type of printing process is merely exemplary of one type of printing process which may be used to form the patterned earplugs.

The externally and internally plasticized polymeric foams disclosed in commonly assigned U.S. Patent No. Re. 29,487 are used as a material of construction for the present earplug. These plasticized polymeric foams are slow recovery foams which are not only comfortable, but also have been shown to deliver high-in-field noise protection at all frequencies. Other suitable materials are disclosed in commonly assigned U.S. Patent No. 5,203,352 to Gardner which discloses temperature-dependent viscoelastic polymeric foam materials. Furthermore, commonly assigned U.S. Patent No. 5,792,998 discloses a dynamically stiff foam material having a low static stiffness and a high dynamic stiffness which provides improved attenuation, wherein for example, the foam component preferably has a dynamic spring constant of at least about 300 pounds per inch and a dynamic loss factor of at least about 0.25.

In one aspect, a suitable foam material is a dynamically stiff polyurethane material. In another aspect, earplugs comprising poly vinyl chloride are suitable. In another aspect, urethane foams disclosed in U.S. Patent No. 4,158,087 to Wood are suitable. It should be understood that the above-described materials are merely exemplary in nature and that other types of materials may be used so long as the earplug may receive a graphic pattern.

By using a printing device, such as an ink jet printer, the earplugs may be graphically patterned and other characters and the like may be formed thereon with relative ease and at reasonable cost. This type of printing process is also very cost effective by virtue of the fact that many earplugs may be processed by the printing device.over a short period of time. The coloring and application of characters and design to the earplugs may also be done using a printing process that produces earplugs having a consistent and uniform appearance. In addition, the present process permits, through high-speed automation, the pattern of the graphic to be repeatable and consistent from earplug to earplug. Furthermore, one aspect of the present printing process permits printing on multiple surfaces of the earplug and allows earplugs to be patterned and uniquely identified with the addition of a contrasting ink or dye which is not limited to specific or single colors. Such patterning may include, but is not limited to, custom logos, printed text, decorative and other patterns which are used for graphic styling or earplug identification.

## Claims

1. A process of producing an earplug (200) the process comprising the steps:
producing the earplug (200) out of a polymeric foam; and forming a pattern (20,40,60,80,100,120) on the earplug including;
orienting the earplug relative to a printing device (210); and
printing the pattern on the earplug using the printing device.

2. The process of claim 1, wherein the printing device (210) comprises an ink jet printer.

3. The process of claim 2, wherein the inkjet printing device forms a dot matrix pattern.

4. The process of claim 1, wherein the printing device (210) comprises a non-impact printing device which is located remote from the earplug (200) when the pattern is formed on the earplug surface and/or an impact type printing device which intimately contacts the earplug surface when the pattern is formed on the foam body.

5. The process of claim 4, wherein the printing device (210) is a PAD printing device or a heat transfer printing device.

6. The process of claim 1 wherein the pattern (20,40,60,80,100,120) includes one or more colours arranged according to a predetermined pattern and/or at least one of a custom logo or printed text.

7. The process of Claim 1, wherein the printing device (210) comprises a plurality of printing devices orientated relative to one another so that a plurality of patterns are formed on the earplug (200).

8. The process of claim 7, wherein the plurality of printing devices are orientated at an angle relative to one another, the angle being 120° or 180° or 90°.

9. The process of claim 1, wherein the earplug (210) is oriented by an alignment device, the alignment device positioning the earplug within or near the printing device (210).

10. The process of claim 9, wherein the alignment device selectively positions the earplug (200) in a plurality of orientations during the printing process.

11. The process of claim 1, wherein the printing device (210) or its at least one nozzle (230) applies a pattern (20,40,60,80,100,120) to the earplug (200) from a plurality of positions.

12. The process of claim 11, wherein the nozzle (230) is associated with a microprocessor (242) and computer unit such that the nozzle selectively applies a pattern (20,40,60,80,100,120) to the earplug (200) from a plurality of orientations.

## Patentansprüche

1. Verfahren zur Herstellung eines Ohrstöpsels (200), wobei das Verfahren die Schritte aufweist:
Herstellen des Ohrstöpsels (200) aus einem polymerischen Schaum; und
Formen eines Musters (20, 40, 60, 80, 100, 120) auf dem Ohrstöpsel (200) einschließlich,
Orientieren des Ohrstöpsels relativ zu einer Druckeinrichtung (210); und
Drucken des Musters auf den Ohrstöpsel unter Verwendung der Druckeinrichtung.

2. Verfahren nach Anspruch 1, wobei die Druckeinrichtung(210) einen Tintenstrahldrucker aufweist.

3. Verfahren nach Anspruch 2, wobei die Tintenstrahldruckeinrichtung ein Punktmatrixmuster bildet.

4. Verfahren nach Anspruch, 1, wobei die Druckeinrichtung eine anschlagfreie oder nicht mechanische Druckeinrichtung aufweist, die entfernt vom Ohrstöpsel (200) angeordnet ist, wenn das Muster auf der Ohrstöpseloberfläche gebildet wird, und/oder eine Druckeinrichtung vom Anschlagtyp aufweist, die in engen Kontakt zur Ohrstöpseloberfläche ist, wenn das Muster auf dem Schaumkörper geformt wird.

5. Verfahren nach Anspruch 4, wobei die Druckeinrichtung (210) eine PAD Druckeinrichtung oder eine Wärmetransferdruckeinrichtung ist.

6. Verfahren nach Anspruch 1, wobei das Muster (20, 40, 60, 80, 100, 120) eine oder mehrere Farben umfasst, die gemäß einem vorbestimmten Muster und/oder gemäß wenigstens einem Kundenlogo oder gedruckten Text angeordnet sind.

7. Verfahren nach Anspruch 1, wobei die Druckeinrichtung (210) eine Vielzahl von Druckeinrichtungen aufweist, die relativ zueinander so orientiert sind, dass eine Vielzahl von Mustern auf dem Ohrstöpsel (200) geformt wird.

8. Verfahren nach Anspruch 7, wobei die Mehrzahl der Druckeinrichtungen in einem Winkel zueinander angeordnet sind, wobei der Winkel 120° oder 180° oder 90 ° beträgt.

9. Verfahren nach Anspruch 1, wobei der Ohrstöpsel (210) von einer Ausrichtungseinrichtung orientiert wird, wobei die Ausrichtungseinrichtung den Ohrstöpsel innerhalb oder nahe der Druckeinrichtung (210) positioniert.

10. Verfahren nach Anspruch 9, wobei die Ausrichtungseinrichtung wahlweise den Ohrstöpsel (200) in einer Vielzahl von Orientierungen während des Druckprozesses positioniert.

11. Verfahren nach Anspruch 1, wobei die Druckeinrichtung (210) oder seine wenigstens eine Düse (230) ein Muster (20, 40, 60, 80, 100, 120) auf dem Ohrstöpsel (200) aus einer Vielzahl von Stetlungen aufbringt.

12. Verfahren nach Anspruch 11, wobei der Düse (230) ein Mikroprozessor (242) und eine Computereinheit so zugeordnet ist, dass die Düse wahlweise ein Muster (20, 40, 60, 80, 100, 120) auf den Ohrstöpsel (200) aus einer Vielzahl von Orientierungen aufbringt.

## Revendications

1. Procédé pour produire un bouchon d'oreille (200), le procédé comportant les étapes consistant à :
produire le bouchon d'oreille (200) en mousse polymérique ; et former un motif (20, 40, 60, 80, 100, 120) sur le bouchon d'oreille en incluant les étapes consistant à :
orienter le bouchon d'oreille par rapport à un dispositif d'impression (210) ; et
imprimer le motif sur le bouchon d'oreille en utilisant le dispositif d'impression.

2. Procédé selon la revendication 1, dans lequel le dispositif d'impression (210) comporte une imprimante à jet d'encre.

3. Procédé selon la revendication 2, dans lequel le dispositif d'impression à jet d'encre forme un motif de matrice de points.

4. Procédé selon la revendication 1, dans lequel le dispositif d'impression (210) comporte un dispositif d'impression sans impact qui est situé à distance du bouchon d'oreille (200) lorsque le motif est formé sur la surface du bouchon d'oreille et/ou un dispositif d'impression de type à impact qui vient en contact intime avec la surface du bouchon d'oreille lorsque le motif est formé sur le corps en mousse.

5. Procédé selon la revendication 4, dans lequel le dispositif d'impression (210) est un dispositif d'impression PAD ou un dispositif d'impression par transfert de chaleur.

6. Procédé selon la revendication 1, dans lequel le motif (20, 40, 60, 80, 100, 120) inclut une ou plusieurs couleurs disposées selon un motif prédéterminé et/ou au moins une couleur d'un logo personnalisé ou d'un texte imprimé.

7. Procédé selon la revendication 1, dans lequel le dispositif d'impression (210) comporte une pluralité de dispositifs d'impression orientés les uns par rapport aux autres de telle sorte qu'une pluralité de motifs soient formés sur le bouchon d'oreille (200).

8. Procédé selon la revendication 7, dans lequel la pluralité de dispositifs d'impression sont orientés en formant un angle les uns par rapport aux autres, l'angle étant égal à 120° ou à 180° ou à 90°.

9. Procédé selon la revendication 1, dans lequel le bouchon d'oreille (210) est orienté par un dispositif d'alignement, le dispositif d'alignement positionnant le bouchon d'oreille à l'intérieur ou près du dispositif d'impression (210).

10. Procédé selon la revendication 9, dans lequel le dispositif d'alignement positionne de manière sélective le bouchon d'oreille (200) dans une pluralité d'orientations pendant le procédé d'impression.

11. Procédé selon la revendication 1, dans lequel le dispositif d'impression (210) ou sa au moins une buse (230) applique un motif (20, 40, 60, 80, 100, 120) sur le bouchon d'oreille (200) à partir d'une pluralité de positions.

12. Procédé selon la revendication 11, dans lequel la buse (230) est associée à un microprocesseur (242) et à une unité informatique de telle sorte que la buse applique de manière sélective un motif (20, 40, 60, 80, 100, 120) sur le bouchon d'oreille (200) à partir d'une pluralité d'orientations.
